# EUROPEAN PATENT SPECIFICATION

(11) **EP 4 151 731 B1**
(45) Date of publication and mention of the grant of the patent: **29.04.2026**
(21) Application number: 21886051.8
(22) Date of filing: 21.10.2021
(51) Int. Cl.: C12N 15/29, C07K 14/415, C12N 1/15, B60C 1/00, C12N 1/19, C12N 1/21, C12N 5/10, C12N 9/10, C12N 15/70, C12P 5/02, C12P 5/00, C12P 21/02

(54) **PROTEIN COMPOSITION HAVING ISOPRENE POLYMERIZATION ACTIVITY AND USE THEREOF**
PROTEINZUSAMMENSETZUNG MIT ISOPRENPOLYMERISATIONSAKTIVITÄT UND VERWENDUNG DAVON
COMPOSITION PROTÉIQUE AYANT UNE ACTIVITÉ DE POLYMÉRISATION DE L'ISOPRÈNE ET SON UTILISATION

(30) Priority: 30.10.2020 JP 2020183088
(43) Date of publication of application: 22.03.2023
(73) Proprietor: Sumitomo Riko Company Limited, Komaki-shi, Aichi 485-8550 (JP)
(72) Inventor: IMAI, Shunsuke, Komaki-shi, Aichi 485-8550 (JP); YONEYAMA, Fuminori, Komaki-shi, Aichi 485-8550 (JP); WAKISAKA, Osamu, Komaki-shi, Aichi 485-8550 (JP)
(74) Representative: Hoffmann Eitle
(86) International application number: PCT/JP2021/038921
(87) International publication number: WO 2022/091932

(56) References cited:
- WO-A1-2012/148253
- WO-A1-2017/002818
- JP-A- 2017 014 361
- US-A1- 2018 171 364
- US-A1- 2018 371 501
- US-A1- 2019 323 039
- CHERIAN SAM ET AL: "Natural rubber biosynthesis in plants, the rubber transferase complex, and metabolic engineering progress and prospects", PLANT BIOTECHNOLOGY JOURNAL, vol. 17, no. 11, 26 June 2019 (2019-06-26), GB, pages 2041 - 2061, XP093061298, ISSN: 1467-7644, Retrieved from the Internet <URL:https://onlinelibrary.wiley.com/doi/full-xml/10.1111/pbi.13181> DOI: 10.1111/pbi.13181
- MINAMI MATSUI: "Rubber Genome & Transcriptome Database", 27 January 2017 (2017-01-27), XP002811247, Retrieved from the Internet <URL:http://matsui- lab.riken.jp/rubber/home.html> [retrieved on 20240320]
- MAKITA YUKO ET AL: "Large-scale collection of full-length cDNA and transcriptome analysis in Hevea brasiliensis", DNA RESEARCH, 27 January 2017 (2017-01-27), JP, pages dsw056, XP093143675, ISSN: 1340-2838, Retrieved from the Internet <URL:doi.10.1093/dnares/dsw056> [retrieved on 20240320], DOI: 10.1093/dnares/dsw056
- DATABASE UNIPROT [online] 1 June 2003 (2003-06-01), ANONYMOUS: "SubName: Full=REF-like stress related protein 2 {ECO:0000313|EMBL:AAP46160.1}; DE SubName: Full=Small rubber particle protein 117 {ECO:0000313|EMBL:AMO43677.1};", XP055927079, retrieved from NCBI Database accession no. Q84T88
- ISHIDA TATSUHIRO, KIWADA HIROSHI: " Interaction of Liposomes with Cell Membrane", MEMBRANE, vol. 32, no. 1, 1 January 2007 (2007-01-01), pages 18 - 24, XP055927060
- DATABASE UNIPROT ANONYMOUS : "SubName: Full=Rubber elongation factor 175 {ECO:0000313|EMBL:AMO43675.1};", XP055927073, retrieved from IBIS
- DATABASE UNIPROT ANONYMOUS : "SubName: Full=REF-like stress related protein 2 {ECO:0000313|EMBL:AAP46160.1}; DE SubName: Full=Small rubber particle protein 117 {ECO:0000313|EMBL:AMO43677.1};", XP055927079, retrieved from NCBI
- THUONG NGHIEM THI, DUNG TRAN ANH, YUSOF NURUL HAYATI, KAWAHARA SEIICHI: "Structure of natural rubber", POLYMER, ELSEVIER, AMSTERDAM, NL, vol. 195, 8 May 2020 (2020-05-08), AMSTERDAM, NL, pages 417 - 423, XP055927064, ISSN: 0032-3861, DOI: 10.1016/j.polymer.2020.122444

## Description

### Field

This invention relates to lipid membrane structures comprising protein compositions having isoprene polymerization activity and uses thereof, cells containing the same, and methods of producing isoprene polymer compounds using the same.

### Background

Natural rubber is a substance composed mainly of cis-polyisoprene contained in sap of the rubber tree. It is widely used in various fields such as the automotive and construction industries, and its demand is expanding worldwide. Since natural rubber is an agricultural crop collected from rubber trees, its supply is unstable, and there are problems such as inconsistent quality, potentially causing a serious risk in business. For this reason, attempts are being made to develop technology to artificially produce natural rubber.

Patent Literature 1 and Non-Patent Literature 1 describe methods of producing polyisoprenoids. Each of the method involves a binding process of binding a rubber particle to each of gene expression products of predetermined proteins (HRT1, HRTBP and REF) involved in natural rubber biosynthesis in vitro.

### Citation List

### Patent Literature

[Patent Literature 1] Japanese Patent No. 6586693

### Non Patent Literature

[Non-Patent Literature 1] Yamashita S.et al. eLife 2016; 5: e19022. doi: 10.7554/eLife.19022

**Cherian S. et al. (2019) relates to natural rubber biosynthesis in plants, the rubber transferase complex, and metabolic engineering progress and prospects.**

US2018/171364 **relates to a production method for polyisoprenoid, as well as a vector, transgenic plant, production method for pneumatic tire, and a production method for rubber product .**

US 2019/323039 **relates to a method for producing polyisoprenoid, transformed plant, method for producing pneumatic tire and method for producing rubber product.**

### Summary

### Technical Problem

The arts described in Patent Literature 1 and Non-Patent Literature 1 were insufficient to produce enough amount of polyisoprenoids for practical use. According to this technology, it is necessary to collect rubber particles from plants, which makes the process of collecting rubber particles complicated, and it is difficult to stabilize the quality of rubber particles because they are extracted from plants.

The present invention provides a means for efficiently producing natural rubber and stably providing the same, and is aimed at stably providing a rubber resource. Specifically, the present invention is defined in the appended claims. Also disclosed is the following.

### Solution to Problem

[1] A protein composition that exhibits an isoprene polymerization activity, the protein composition comprising a protein(s) (B) and either one of proteins (A-1) and (A-2):
   (A-1) one or more protein(s) selected from a protein(s) comprising an amino acid sequence of SEQ ID NO: 6 and a protein(s) that comprises an amino acid sequence having 90% or more identity with the amino acid sequence of SEQ ID NO: 6 and exhibits the same activity as CPT6;
   (A-2) one or more protein(s) selected from a protein(s) comprising an amino acid sequence of SEQ ID NO: 8 and a protein(s) that comprises an amino acid sequence having 90% or more identity with the amino acid sequence of SEQ ID NO: 8 and exhibits the same activity as CPT7; and
   (B) one or more protein(s) selected from a protein(s) comprising an amino acid sequence of SEQ ID NO: 10 and a protein(s) that comprises an amino acid sequence having 90% or more identity with the amino acid sequence of SEQ ID NO: 10 and exhibits the same activity as CPTL.
[2] The composition according to [1], wherein the composition fulfills at least one of the followings:
   the protein (A-1) encoded by one or more polynucleotide(s) (a-1) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
   the protein (A-2) encoded by one or more polynucleotide(s) (a-2) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
   the protein (B) encoded by one or more polynucleotide(s) (b) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein exhibiting the same activity as CPTL.
[3] The composition according to [1] or [2], further comprising an REF family protein, an SRPP family protein, and a CPT family protein other than CPT6, 7 and CPTL.
[4] The composition according to [1], further comprising at least one of the followings:
   (C) one or more protein(s) selected from a protein(s) comprising an amino acid sequence of SEQ ID NO: 2 and a protein(s) that comprises an amino acid sequence having 90% or more identity with the amino acid sequence of SEQ ID NO: 2 and exhibits the same activity as CPT1;
   (D) one or more protein(s) selected from a protein(s) comprising an amino acid sequence of SEQ ID NO: 4 and a protein(s) that comprises an amino acid sequence having 90% or more identity with the amino acid sequence of SEQ ID NO: 4 and exhibits the same activity as CPT2;
   (E) one or more protein(s) selected from a protein(s) comprising an amino acid sequence of SEQ ID NO: 12 and a protein(s) that comprises an amino acid sequence having 90% or more identity with the amino acid sequence of SEQ ID NO: 12 and exhibits the same activity as REF1;
   (F) one or more protein(s) selected from a protein(s) comprising an amino acid sequence of SEQ ID NO: 14 and a protein(s) that comprises an amino acid sequence having 90% or more identity with the amino acid sequence of SEQ ID NO: 14 and exhibits the same activity as REF2;
   (G) one or more protein(s) selected from a protein(s) comprising an amino acid sequence of SEQ ID NO: 16 and a protein(s) that comprises an amino acid sequence having 90% or more identity with the amino acid sequence of SEQ ID NO: 16 and exhibits the same activity as REF8; and
   (H) one or more protein(s) selected from a protein(s) comprising an amino acid sequence of SEQ ID NO: 18 and a protein(s) that comprises an amino acid sequence having 90% or more identity with the amino acid sequence of SEQ ID NO: 18 and exhibits the same activity as SRPP1.
[5] The composition according to [4], wherein the composition fulfills at least one of the followings:
   the protein (C) encoded by one or more polynucleotide(s) (c) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
   the protein (D) encoded by one or more polynucleotide(s) (d) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
   the protein (E) encoded by one or more polynucleotide(s) (e) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
   the protein (F) encoded by one or more polynucleotide(s) (f) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
   the protein (G) encoded by one or more polynucleotide(s) (g) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
   the protein (H) encoded by one or more polynucleotide(s) (h) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.
[6] The composition according to any one of [1] to [5], wherein at least one of the proteins (A) through (H) is a protein derived from a rubber tree.
[7] A lipid membrane structure comprising the composition according to any one of [1] to [6] and a phospholipid.
[8] The structure according to [7], wherein the structure is a lipid bilayered membrane structure.
[9] The structure according to [7] or [8], wherein the structure is a proteoliposome.
[10] A method for producing the lipid membrane structure according to any one of [7] to [9], the method comprising expressing the polynucleotide encoding the protein constituting the composition according to any one of [1] to [6] in a cell-free protein production system in the presence of a raw material containing a phospholipid.
[11] The method for producing the lipid membrane structure according to [10], wherein the polynucleotide encoding the protein constituting the composition according to any one of [1] to [6] comprises (b) and either one of (a-1) and (a-2):
   (a-1) one or more polynucleotide(s) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
   (a-2) one or more polynucleotide(s) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
   (b) one or more polynucleotide(s) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein exhibiting the same activity as CPTL.
[12] The method for producing the lipid membrane structure according to [10] or [11], wherein the polynucleotide is the polynucleotide encoding the protein constituting the composition according to [3] or [4], and comprises at least one of the followings:
   (c) one or more polynucleotide(s) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
   (d) one or more polynucleotide(s) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
   (e) one or more polynucleotide(s) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
   (f) one or more polynucleotide(s) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
   (g) one or more polynucleotide(s) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
   (h) one or more polynucleotide(s) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.
[13] The method for producing the lipid membrane structure according to any one of [10] to [12], wherein the raw material containing the phospholipid contains a liposome, and wherein the lipid membrane structure is a proteoliposome.
[14] A cell expressing a protein constituting the composition of any one of [1] to [5].
[15] The cell according to [14], comprising an expression unit containing a polynucleotide that encodes each of the proteins in the protein composition of any one of [1] to [6].
[16] The cell according to [14] or [15], wherein the polynucleotide comprises (b) and either one of (a-1) and (a-2):
   (a-1) one or more polynucleotide(s) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
   (a-2) one or more polynucleotide(s) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
   (b) one or more polynucleotide(s) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein exhibiting the same activity as CPTL.
[17] The cell according to any one of [14] to [16], wherein the polynucleotide is the polynucleotide encoding the protein constituting the composition according to [3] or [4], and further comprises at least one of the followings:
   (c) one or more polynucleotide(s) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
   (d) one or more polynucleotide(s) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
   (e) one or more polynucleotide(s) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
   (f) one or more polynucleotide(s) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
   (g) one or more polynucleotide(s) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
   (h) one or more polynucleotide(s) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.
[18] The cell according to any one of [14] to [17], wherein the expression unit is a heterologous expression unit.
[19] The cell according to [14], comprising the lipid membrane structure according to [9].
[20] The method for producing the cell according to [14], comprising interacting the cell with the lipid membrane structure according to [9].
[21] A method for producing an isoprene polymer compound, the method comprising performing an isoprene polymerization reaction using at least one selected from the composition according to any one of [1] to [6], the structure according to any one of [7] to [9], and the cell according to any one of [14] to [19].
[22] The production method according to [21], wherein the isoprene polymerization reaction is performed using a low molecular weight allyl compound as a substrate.
[23] A kit for producing an isoprene polymer compound, the kit comprising:
   a low molecular weight allyl compound; and
   at least one selected from the composition according to any one of [1] to [6], the structure according to any one of [7] to [9], and the cell according to any one of [14] to [19].
[24] A method for producing a rubber, the method comprising producing the rubber using an isoprene polymer compound produced by the production method according to [21] or [22].

### Advantageous Effects of Invention

The present invention is expected to efficiently produce and stably provide natural rubber, thereby stably providing a rubber resource for which demand is growing worldwide.

### Description of aspects

### [Protein composition]

### (Isoprene polymerization activity)

A protein composition of the present disclosure exhibits isoprene polymerization activity. The isoprene polymerization activity typically refers to an activity that directly or indirectly promotes an isoprene polymerization reaction to produce an isoprene polymer compound. The isoprene polymerization reaction is typically a polymerization reaction of isoprene in living organisms. Examples of substrates include low molecular weight allyl compounds such as isopentenyl diphosphate (IPP), farnesyl diphosphate (FPP), dimethylallyl diphosphate (DMAPP), geranyl diphosphate (GPP) and geranylgeranyl diphosphate (GGPP).

Examples of the organisms include rubber trees (plants capable of producing natural rubber) such as plants of the genus *Hevea* (e.g., para rubber tree *(Hevea brasiliensis), Hevea benthamiana, Hevea guianensis),* plants of the genus *Ficus* (e.g., Indian rubber tree *(Ficus elastica),* oak rubber tree *(Ficus lyrata)* and Benjamin rubber tree *(Ficus benjamina),* and the organism is not limited to the above plants. Examples of the organisms also include plant cells, animal cells and microorganisms.

### (Protein constituting protein composition) (Proteins (A) and (B))

The protein composition contains a protein(s) (B) and at least one of proteins (A-1) and (A-2):
(A-1) one or more protein(s) selected from a protein(s) comprising an amino acid sequence of SEQ ID NO: 6 and a protein(s) that comprises an amino acid sequence having 90% or more identity with the amino acid sequence of SEQ ID NO: 6 and exhibits the same activity as CPT6;
(A-2) one or more protein(s) selected from a protein(s) comprising an amino acid sequence of SEQ ID NO: 8 and a protein(s) that comprises an amino acid sequence having 90% or more identity with the amino acid sequence of SEQ ID NO: 8 and exhibits the same activity as CPT7; and
(B) one or more protein(s) selected from a protein(s) comprising an amino acid sequence of SEQ ID NO: 10 and a protein(s) that comprises an amino acid sequence having 90% or more identity with the amino acid sequence of SEQ ID NO: 10 and exhibits the same activity as CPTL.

The amino acid sequences of SEQ ID NO: 6, 8 and 10 are amino acid sequences of cis-prenyltransferase (CPT) 6, CPT7 and CPTL, respectively, derived from para rubber tree *(Hevea brasiliensis*).

As well, the proteins (A-1), (A-2) and (B) may be proteins encoded by polynucleotides (a-1), (a-2) and (b) encoding (A-1), (A-2) and (B), respectively:
(a-1) one or more polynucleotide(s) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
(a-2) one or more polynucleotide(s) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
(b) one or more polynucleotide(s) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein exhibiting the same activity as CPTL.

The nucleic acid sequences of SEQ ID NO: 5, 7 and 9 are full-length nucleic acid sequences of CPT6, CPT7 and CPTL, respectively, derived from para rubber tree *(Hevea brasiliensis).*

### (Other proteins)

The protein composition may contain (an)other protein(s) in addition to the proteins (A-1), (A-2) and (B). Examples of the other protein(s) include proteins responsible for the isoprene polymerization reaction itself in vivo and proteins known to assist in the polymerization reaction. Examples of the former include CPT families other than CPT6, CPT7 and CPTL. Examples of the latter includes at least one selected from a rubber elongation factor (REF) family and a small rubber particle protein (SRPP) family. Among them, at least one of proteins (C) to (H) is preferred:
(C) one or more protein(s) selected from a protein(s) comprising an amino acid sequence of SEQ ID NO: 2 and a protein(s) that comprises an amino acid sequence having 90% or more identity with the amino acid sequence of SEQ ID NO: 2 and exhibits the same activity as CPT1;
(D) one or more protein(s) selected from a protein(s) comprising an amino acid sequence of SEQ ID NO: 4 and a protein(s) that comprises an amino acid sequence having 90% or more identity with the amino acid sequence of SEQ ID NO: 4 and exhibits the same activity as CPT2;
(E) one or more protein(s) selected from a protein(s) comprising an amino acid sequence of SEQ ID NO: 12 and a protein(s) that comprises an amino acid sequence having 90% or more identity with the amino acid sequence of SEQ ID NO: 12 and exhibits the same activity as REF1;
(F) one or more protein(s) selected from a protein(s) comprising an amino acid sequence of SEQ ID NO: 14 and a protein(s) that comprises an amino acid sequence having 90% or more identity with the amino acid sequence of SEQ ID NO: 14 and exhibits the same activity as REF2;
(G) one or more protein(s) selected from a protein(s) comprising an amino acid sequence of SEQ ID NO: 16 and a protein(s) that comprises an amino acid sequence having 90% or more identity with the amino acid sequence of SEQ ID NO: 16 and exhibits the same activity as REF8; and
(H) one or more protein(s) selected from a protein(s) comprising an amino acid sequence of SEQ ID NO: 18 and a protein(s) that comprises an amino acid sequence having 90% or more identity with the amino acid sequence of SEQ ID NO: 18 and exhibits the same activity as SRPP1.

The amino acid sequences of SEQ ID NO: 2, 4, 12, 14, 16 and 18 include mature proteins of CPT1, CPT2, REF1, REF2, REF8 and SRPP1, respectively, derived from para rubber tree

### (Hevea brasiliensis).

As well, the proteins (C) to (H) may be proteins encoded by polynucleotides (c) to (h) encoding (C) to (H), respectively:
(c) one or more polynucleotide(s) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
(d) one or more polynucleotide(s) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
(e) one or more polynucleotide(s) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
(f) one or more polynucleotide(s) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
(g) one or more polynucleotide(s) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
(h) one or more polynucleotide(s) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.

The nucleic acid sequences of SEQ ID NO: 1, 3, 11, 13, 15 and 17 are full-length nucleic acid sequences of CPT1, CPT2, REF1, REF2, REF8 and SRPP1, respectively, derived from para rubber tree *(Hevea brasiliensis).*

The protein composition preferably contains two, three, four or five or more of the proteins (C) through (H), more preferably contains a combination of at least one of (C) and (D) and at least one of (E) through (H), and still more preferably contains all.

### (Identity %)

The sequence identities of amino acid and nucleotide may be 90%, 91%, 92%, 93%, 94%, 95%, 96%, 97%, 98% or 99% or more. The identity can be determined under default conditions using NCBI BLAST (see http://www.ncbi.nlm.nih.gov), for example.

### (Activity possessed by mutant protein)

The term "exhibiting same activity" as each protein means exhibiting 50% or more, 60% or more, 70% or more, 80% or more, 90% or more, 95% or more, or 100% of the activity of each protein measured under the same condition.

### (Mutation)

The above proteins may have mutations introduced at a site in a catalytic domain and at a site outside the catalytic domain, as long as they maintain same activity. A person skilled in the art can determine the position of the amino acid residue to which the mutation may be introduced in the protein while maintaining the aimed activity. For example, a person skilled in the art can 1) compare the amino acid sequences (e.g., the amino acid sequence represented by SEQ ID NO: 6 or 8, and the amino acid sequences of other proteins involved in the isoprene polymerization reaction) of a plurality of proteins having similar activities, 2) identify a relatively conserved region(s) and a region(s) not conserved, and then 3) predict regions capable and incapable of serving important roles in function from the relatively conserved region(s) and the region(s) not conserved in order to recognize the correlation in structure and/or function and determine a portion(s) to which the mutation can be introduced.

The mutation introduced into the protein can be a substitution of an amino acid residue for another amino acid residue, preferably a substitution with an amino acid residue having a similar side chain. Examples of classification of amino acids by amino acid residues with similar side chains include: amino acids with basic side chains such as lysine, arginine and histidine; amino acids with acidic side chains such as aspartic acid and glutamic acid; amino acids with uncharged polar side chains such as asparagine, glutamine, serine, threonine, tyrosine and cysteine; amino acids with nonpolarized side chains such as glycine, alanine, valine, leucine, isoleucine, proline, phenylalanine, methionine and tryptophan; amino acids with beta-branched side chains such as leucine, valine and isoleucine; amino acids with aromatic side chains such as tyrosine and phenylalanine, tryptophan and histidine; amino acids with hydroxyl group-containing side chains (e.g., alcohol, phenoxy group-containing side chains) such as serine, threonine and tyrosine; and amino acids with sulfur-containing side chains such as cysteine and methionine.

The nucleic acid sequences may be modified for codon optimization. The codon optimization in this description refers to modification involving optimization of an expression in a targeted certain cell without changing the sequence of the encoded polypeptide. A person skilled in the art can understand a frequency of codon usage in the cell to be transduced and easily determine the sequence obtained after optimization by utilizing genetic code degeneracy. Examples of the target cells include microorganisms such as *E. coli,* plant cells and animal cells.

### (Protein modification)

Each of the proteins constituting the protein composition may have a purification tag at the C-terminus. Examples of the purification tag include histidine tags; HA, FLAG, V5 and myc epitopes; chitin binding protein (CBP); maltose binding protein (MBP); glutathione-S-transferase (GST); and Strep tags. The protein composition may contain other factors directly or indirectly involved in the isoprene polymerization reaction in addition to those listed above.

### (Lipid membrane structure)

The lipid membrane structure of the present disclosure is a structure including lipid membranes that contains the above protein composition. The use of the lipid membrane structure enables the protein composition to facilitate the isoprene polymerization activity, and achieve efficient isoprene polymerization.

The lipid membrane can be a monolayered membrane or a bilayered membrane. Examples of the structure of the monolayered membrane include rubber particles (particles extracted from latex derived from the rubber tree). Examples of the structure of the bilayered membrane include proteoliposomes and artificial lipid membrane models. The artificial lipid membrane models may be composed of either one or both of natural lipids and synthetic lipids, and are not particularly limited. The lipid membrane structure is preferably the structure of the bilayered membrane, more preferably proteoliposome.

### (Proteoliposome)

In this description, proteoliposome refers to an artificial vesicle in which a protein is encapsulated or bound to a portion of the lipid bilayer of liposome. In this description, liposome refers to an artificial vesicle in which an aqueous component (usually water) is trapped in a space formed by the lipid bilayer membrane. The lipid bilayer membrane is typically composed of a lipid(s) such as phospholipid. Example of the phospholipid include phosphatidylethanolamine, phosphatidylcholine, phosphatidylserine, cardiolipin, or combinations thereof. Example of the lipid other than phospholipid include triacylglycerol, waxes, sphingolipids and sterols and fatty acid esters thereof, or combinations thereof. The lipid constituting the lipid bilayer membrane preferably include a biogenic phospholipid, more preferably include soybean-derived phospholipid.

The size of liposome is not particularly limited, but typically 50 to 300 nm in diameter. Liposome may be naturally occurring or synthesized, and is preferably the former, more preferably liposome derived from a plant such as soybean. The synthesized liposome may be polyalkylene glycol-based liposome, for example.

The form of the protein composition in the membrane lipid structure is not particularly limited, but presumed to be a form in which the protein composition is bound to hydrophilic groups of the phospholipids on the liposome surface (some may be embedded in the membrane interior) in the case of liposome.

### (Production method)

A method for producing the membrane lipid structure may be a method involving expression of the polynucleotide encoding the protein constituting the composition in a cell-free protein production system in the presence of source materials including the phospholipids, for example.

The source materials including phospholipids include the liposome if the membrane structure is the proteoliposome. The liposome is preferably the naturally occurring liposome, as described above, and preferably the liposome derived from the plant such as soybean.

The method for producing the proteoliposome preferably includes expressing an expression unit containing the polynucleotide in the cell-free protein production system in the presence of liposome.

The expression unit contains at least polynucleotides encoding the proteins contained in the protein composition. The polynucleotides refer to polynucleotides encoding the proteins constituting the protein composition, include (b) and either one of (a-1) and (a-2) described above, and further include at least one of (c) through (h) as required. The expression unit typiclly includes a promoter. This allows a polynucleotide expression product encoded by the polynucleotides to be efficiently produced. Examples of the promoter include tac promoter, lac promoter, trp promoter, trc promoter, T7 promoter, T5 promoter, T3 promoter, and SP6 promoter, and may include an enhancer. The promoter is linked to the polynucleotide in a form capable of expressing it. For example, promoter can be linked in an upstream (5') side of the code sequence. The expression unit can be DNA or RNA, but is preferably DNA. The expression unit may contain factors such as terminators, ribosome binding sites, drug resistance genes, RNA processing signals, sequences that improve translation efficiency, sequences that improve protein stability, and sequences that improve protein secretion.

The cell-free protein production system is preferably performed using a cell extract (e.g., wheat germ extract, *E. coli* extract, rabbit reticulocyte extract and insect cell extract) by a bilayer-dialysis method and can be performed using a protein cell-free expression kit commercially available.

It is possible to use another protein building system, such as artificial lipid membrane model, instead of the liposome. The protein composition in the artificial lipid membrane model can be formed according to a conventional method. The artificial lipid membrane model may be composed of either one or both of naturally occurring lipids, synthetic lipids, and is not particularly limited.

### (Cell)

The cell of the present disclosure is a cell that expresses each of the proteins that make up the protein composition (proteins (A) and (B), and at least one protein of (C) through (H), as required). Examples of the cell include cells of microorganisms such as *E. coli* and yeast, plant cells, and animal cells.

The cell preferably contains the expression unit containing the polynucleotide(s). The expression unit is same as those explained for the proteoliposome. The expression unit containable in cells is preferably a heterologous expression unit. The heterologous expression unit in this description refers to an expression unit in which at least one of the polynucleotides encoding each of the proteins and the promoter normally contained is not intrinsic to the host cell. Preferably, both are not intrinsic to the host cell.

A method for producing the cell may be a method of transforming with an expression vector containing an expression unit that contains a polynucleotide encoding each of the proteins constituting the above protein compositions, for example. Since the protein composition contains at least two proteins as described above, the expression vector may be one (containing an expression unit containing polynucleotides encoding all proteins) or two or more (containing an expression unit containing polynucleotides for each or every few). The expression vector may be an integrative vector or a non-integrative vector. In the expression vector, the polynucleotide(s) can be placed under the control of a constitutive promoter or an inducible promoter.

A host can be transformed with the expression vector by one or more well-known method(s). Examples of such methods include calcium phosphate method, liposome method, DEAE dextran method, electroporation method, and particle gun (gene gun) method. The transformation can be performed by a method of infecting for the introduction in bacteria cells with use of a phage vector in addition to the plasmid vector.

The cell may be a cell containing the proteoliposome described above, as well. The proteoliposome is preferably prepared from the naturally occurring liposome. This facilitates interaction with the cell membrane. The cell containing proteoliposome can be produced by interacting the proteoliposome with the cell membrane. Examples of the methods for the interaction include adsorption or binding of the liposome to the cell surface, uptake of the liposome into the cell (endocytosis or phagocytosis), and fusion of the lipid bilayer membrane of the liposome with the cell membrane.

### [method for producing isoprene polymerization compound]

The above protein composition, the proteoliposome and the cells can be used to produce the isoprene polymerization compound.

In the production of isoprene polymerization compound, a low molecular weight allylic compound is used as a substrate to perform the isoprene polymerization reaction. Examples of the low molecular weight allylic compound include isoprenyl diphosphate and farnesyl diphosphate, both of which are preferably used. Conditions such as reaction temperature and reaction time can be set as appropriate. A typical isoprene polymerization compound is cis-polyisoprene, which is useful as a raw material of the rubber. Other examples of the isoprene polymerization compound include dolichol, polyprenol and derivatives thereof, which exhibit physiological activities (e.g., immune activity, antiviral activity and antioxidant activity) in the body and are therefore useful as materials for pharmaceuticals and health foods. It can be used as an additive (e.g., plasticizer, compatibilizer) for rubber products, as well.

### [Kit for the production of isoprene polymerization compound]

The above protein composition, proteoliposome and the cells can be used as a kit for the production of isoprene polymerization compound. The Kit typically contains the low molecular weight allyl compound as the substrate. The Kit may further contain a reaction vessel and needed reagents.

### [Method for producing rubber]

The isoprene polymer compound obtained by the above isoprene polymer compound production method can be used in the production of rubber. The procedure and conditions for producing the rubber from the isoprene polymer compound may be in conformity with the conventional method, such as a method of kneading the isoprene polymer compound, crosslinking and then molding, for example. Examples of the additives that can be used as required include reinforcing agents, silane coupling agents, fillers, vulcanizing agents, vulcanization accelerators, vulcanization accelerator auxiliary agents, oils, curing resins, waxes, anti-aging agents and coloring agents. These additives are added at appropriate stages. The resulting rubber can be used for various applications such as tires, construction materials, sporting goods, and automotive parts.

### [Examples]

### Examples 1 to 11

### (1) Acquisition of natural rubber biosynthesis protein gene

Seven proteins (CPT1, CPT2, CPTL, REF1, REF2, REF8 and SRPP1) capable of being produced in latex at high yields were selected from "Rubber Database" (http://Matsui-lab.riken.jp/rubber/home.html). Two proteins (CPT6 and CPT7) were also selected from the phylogenetic analysis.

PCR was carried out using plasmids containing genes encoding the proteins or cDNA thereof as templates to provide genes of CPT1, CPT2, CPT6, CPT7, CPTL, REF1, REF2, REF8 and SRPP1. PCR was performed according to the instructions for PrimeSTAR Max DNA Polymerase (Takara Bio Inc.) or KOD One PCR Master Mix (TOYOBO CO., LTD.). A restriction enzyme digestion site and a His tag sequence were added to the primer as required.

The following primer sets were used for the acquisition of the genes of CPT1, CPT2, CPT6, CPT7, CPTL, REF1, REF2, REF8 and SRPP1: a set of primers 1 and 2, a set of primers 3 and 4, a set of primers 5 and 6, a set of primers 7 and 8, a set of primers 9 and 10, a set of primers 11 and 12, a set of primers 13 and 14, a set of primers 15 and 16, and a set of primers 17 and 18, respectively.

### (Primer set for acquisition of CPT1 gene)

Primer 1: 5'-atggaattatacaacggtg-3' (SEQ ID NO: 19)
Primer 2: 5'-atctcgagttaatgatgatgatgatgatgttttaag-3' (SEQ ID NO: 20)

### (Primer set for acquisition of CPT2 gene)

Primer 3: 5'-atggaaatatatacgggtcag-3' (SEQ ID NO: 21)
Primer 4: 5'-atctcgagttaatgatgatgatgatgatgttttaaatattc-3' (SEQ ID NO: 22)

### (Primer set for acquisition of CPT6 gene)

Primer 5: 5'-atggaaaaacatagcagtag-3' (SEQ ID NO: 23)
Primer 6: 5'-atctcgagttatataactgatgctttttc-3' (SEQ ID NO: 24)

### (Primer set for acquisition of CPT7 gene)

Primer 7: 5'-atgcaatccttgcacttg-3' (SEQ ID NO: 25)
Primer 8: 5'-atctcgagttatgtaagtcgtctaccatag-3' (SEQ ID NO: 26)

### (Primer set for acquisition of CPTL gene)

Primer 9: 5'-atggatttgaaacctgg-3' (SEQ ID NO: 27)
Primer 10: 5'-atctcgagttaatgatgatgatgatgatgtgtac-3' (SEQ ID NO: 28)

### (Primer set for acquisition of REF1 gene)

Primer 11: 5'-atggctgaaggtgaagaagaggtgaatatc-3' (SEQ ID NO: 29)
Primer 12: 5'-atctcgagtcacccatctccatatagcac-3' (SEQ ID NO: 30)

### (Primer set for acquisition of REF2 gene)

Primer 13: 5'-atggctgaagacgaagacaaccaacaag-3' (SEQ ID NO: 31)
Primer 14: 5'-atctcgagtcaattctctccataaaacac-3' (SEQ ID NO: 32)

### (Primer set for acquisition of REF8 gene)

Primer 15: 5'-atggctgaagggaaagaaaacgagaatttc-3' (SEQ ID NO: 33)
Primer 16: 5'-atctcgagttactctgcactttccttcac-3' (SEQ ID NO: 34)

### (Primer set for acquisition of SRPP1 gene)

Primer 17: 5'-atggctgaagaggtggaggaagagaggc-3' (SEQ ID NO: 35)
Primer 18: 5'-atctcgagttatgatgcctcatctccaaac-3' (SEQ ID NO: 36)

For each gene obtained by the method described above, the sequence was identified, and the full-length nucleic acid sequence and amino acid sequence were identified. SEQ ID NO: 1, 3, 5, 7, 9, 11, 13, 15 and 17 represent nucleic acid sequences of CPT1, CPT2, CPT6, CPT7, CPTL, REF1, REF2, REF8 and SRPP1 genes, respectively. SEQ ID NO: 2, 4, 6, 8, 10, 12, 14, 16 and 18 represent amino acid sequences of CPT1, CPT2, CPT6, CPT7, CPTL, REF1, REF2, REF8 and SRPP1 genes, respectively.

### (2) Preparation of plasmid for cell-free protein synthesis

Each of PCR reaction products of the genes obtained in the above (1) was treated with a restriction enzyme XhoI added to the primer. The combinations listed in Table 1 were selected to be inserted into a cell-free expression vector pEU-E01-MCS treated with EcoRV and XhoI restriction enzymes to prepare plasmids for cell-free protein synthesis.

### (3) Transformation of E. coli

The prepared plasmids were transformed in *E. coli* JM 109. The transformant was cultured in LB agar medium containing ampicillin to select the transformant to which a target plasmid was introduced by colony PCR.

### (4) Preparation of plasmid for cell-free protein synthesis

*E. coli* transformed with a plasmid containing the target gene was cultured in LB liquid medium at 37°C overnight. The bacterial cells were collected for recovery of the plasmid. Plasmids were recovered using the FastGene Plasmid Mini Kit (Nippon Genetics Co., Ltd.). The absence of mutations in the gene sequence inserted in the recovered plasmid was confirmed by sequencing analysis.

### (5) Transformation of E. coli

The plasmid prepared as above was used for transformation of *E. coli* JM109. The transformant was cultured in LB agar medium containing ampicillin.

### (6) Recovery of plasmid for cell-free protein synthesis

*E. coli* transformed with a plasmid containing the target gene was cultured in LB liquid medium at 37°C overnight. The bacterial cells were collected for recovery of the plasmid. The plasmid was recovered using QIAGEN Plasmid Midi Kit (QIAGEN) and prepared to 1 µg/µL.

### (7) Synthesis of membrane protein (proteoliposome) in the presence of liposome by bilayer-dialysis method

A lipid bilayered membrane (liposome: approximately several hundreds nm in diameter) was prepared with use of soybean-derived phospholipids. Cell-free protein synthesis was performed under liposome addition conditions according to the protocol supplied with the ProteoLiposome BD Expression Kit (CellFree Sciences Co., Ltd.). The plasmids for expression of the nine proteins used as templates were prepared to 1 µg/µL and used according to the combinations listed in Table 1 in equivalent ratio so that the total volume was approximately 13 µL. For example, in Example 3, 1.5 µL for each of 9 species, 13.5 µL in total was added to the transcription reaction system. After the synthesis reaction was completed, a simple purification of Proteoliposomes with 9 proteins bound to liposomes was performed according to the protocol provided, and the synthesis was confirmed by SDS-PAGE.

### (8) Measurement of isoprene polymerization activity

The isoprene polymerization activities of cell-free synthesized proteins were determined by the following method.

Tris-HCl (pH 7.5) at a final concentration of 50 mM was mixed with MgCl₂ at a final concentration of 10 mM, DTT at a final concentration of 2 mM, farnesyl diphosphate (FPP) at a final concentration of 10 µM, ¹⁴C-isopentenyldiphosphate (¹⁴C-IPP) (specific activity: 1.48 to 2.22 GBq/mmol) at a final concentration of 50 µM, and 5 µL Proteoliposome to prepare 100 µL of reaction solution. The reaction solution was allowed to react at 30°C for 24 hours.

After the reaction, 200 µL of saturated saline solution was added and the reaction product was extracted with 300 µL of water-saturated n-butanol. 400 µL of 2M NaCl was added to the collected water-saturated n-butanol layer for suspension to collect the water-saturated n-butanol layer. To 50 µL of the collected solution, 5 mL of Clear sol II (Nacalai Tesque, Inc.) was added and mixed well. The mixture solution was used for measurement of radioactivity (dpm) with a liquid scintillation counter (LSC-8000, Aloka Co., Ltd.). The reaction system without addition of Proteoliposome was used as a negative control. Table 1 represents results for Proteoliposome containing various proteins.

### (9) TLC radiogram of isoprene polymerization reaction product

An organic layer was volatilized from the water-saturated n-butanol solution collected as above with centrifugal evaporator. 1.5 mL of a reaction solution was prepared by mixing sodium acetate (pH 5.5) at a final concentration of 100 mM with acid phosphatase at a final concentration of 1 U and 1 mL methanol derived from potato, and then allowed to react at 37°C for 17 hours.

After the reaction, 500 µL of hexane was added for extraction. The resulting hexane layer was concentrated to dryness with centrifugal evaporator. The dried material was redissolved into 10 µL of hexane, and all amount of the solution was spotted onto a TLC plate (Merck, TLC glass plate RP-18F254S 5 x 10 cm). Spots developed by acetone : water = 19 : 1 was subjected to light exposure on an imaging plate and visualized using a scanner (Typhoon FLA9500).

### Comparative example 1 (negative control)

The same operations were performed as in Example described above except that the proteoliposome was not added.

### Comparative Examples 2 to 9 (other than negative control)

Plasmids for cell-free synthesis of the proteins were prepared to 1 µg/µL. The total amount of the plasmid in the reaction was 13.5 µL, and each plasmid was added in the same amount. Thereafter, the same operations were performed as in Example described above.

**[Table 1]**

| **Table 1 Combination of protein and measurement result for radioactivity in Examples** | | | | | | | | | | |
|---|---|---|---|---|---|---|---|---|---|---|
| | **CPT1 (C)** | **CPT2 (D)** | **CPT6 (A-1)** | **CPT7 (A-2)** | **CPTL (B)** | **REF1 (E)** | **REF2 (F)** | **REF8 (G)** | **SRPP1 (H)** | **Radioactivity** |
| **Example 1** | - | - | + | - | + | - | - | - | - | **7655.5** |
| **Example 2** | - | - | - | + | + | - | - | - | - | **5041.5** |
| **Example 3** | + | + | + | + | + | + | + | + | + | **15173.2** |
| **Example 4** | - | + | + | + | + | + | + | + | + | **9449.0** |
| **Example 5** | + | - | + | + | + | + | + | + | + | **6764.0** |
| **Example 6** | + | + | - | + | + | + | + | + | + | **2975.2** |
| **Example 7** | + | + | + | - | + | + | + | + | + | **4660.0** |
| **Example 8** | + | + | + | + | + | - | + | + | + | **8233.7** |
| **Example 9** | + | + | + | + | + | + | - | + | + | **5697.5** |
| **Example 10** | + | + | + | + | + | + | + | - | + | **7280.7** |
| **Example 11** | + | + | + | + | + | + | + | + | - | **7143.3** |
| **Comparative example 1** | - | - | - | - | - | - | - | - | - | **152.3** |
| **Comparative example 2** | + | - | - | - | - | - | - | - | - | **303.4** |
| **Comparative example 3** | - | + | - | - | - | - | - | - | - | **401.6** |
| **Comparative example 4** | - | - | + | - | - | - | - | - | - | **328.1** |
| **Comparative example 5** | - | - | - | + | - | - | - | - | - | **340.8** |
| **Comparative example 6** | + | - | - | - | + | - | - | - | - | **289.7** |
| **Comparative example 7** | + | - | - | - | + | - | + | - | - | **388.1** |
| **Comparative example 8** | + | + | + | + | - | + | + | + | + | **305.5** |
| **Comparative example 9** | + | + | - | - | + | + | + | + | + | **205.6** |

As clearly demonstrated in Table 1, the radioactivity in each Example was higher than that in Comparative examples, revealing that the product (presumed to have a molecular weight of 1,000 to 2,000) was synthesized. The combination of CPT6 and CPTL in Example 1 and the combination of CPT7 and CPTL in Example 2 exhibit higher radioactivities than Comparative examples, demonstrating that each combination exhibits isoprene polymerization activity. Furthermore, other protein combinations in Examples 3 to 11 also exhibited higher radioactivities than Comparative examples. Some of them exhibited higher radioactivities than Examples 1 and 2, demonstrating that the proteins other than CPT6, 7, and CPTL are also capable of promoting the isoprene polymerization activity.

These results reveal that the present invention enables efficient production of natural rubber.
SEQ ID NO: 1: Nucleic acid sequence of CPT1
SEQ ID NO: 2: Amino acid sequence of CPT1
SEQ ID NO: 3: Nucleic acid sequence of CPT2
SEQ ID NO: 4: Amino acid sequence of CPT2
SEQ ID NO: 5: Nucleic acid sequence of CPT6
SEQ ID NO: 6: Amino acid sequence of CPT6
SEQ ID NO: 7: Nucleic acid sequence of CPT7
SEQ ID NO: 8: Amino acid sequence of CPT7
SEQ ID NO: 9: Nucleic acid sequence of CPTL
SEQ ID NO: 10: Amino acid sequence of CPTL
SEQ ID NO: 11: Nucleic acid sequence of REF1
SEQ ID NO: 12: Amino acid sequence of REF1
SEQ ID NO: 13: Nucleic acid sequence of REF2
SEQ ID NO: 14: Amino acid sequence of REF2
SEQ ID NO: 15: Nucleic acid sequence of REF8
SEQ ID NO: 16: Amino acid sequence of REF8
SEQ ID NO: 17: Nucleic acid sequence of SRPP1
SEQ ID NO: 18: Amino acid sequence of SRPP1
SEQ ID NO: 19: Primer 1 (for acquisition of CPT1 gene)
SEQ ID NO: 20: Primer 2 (for acquisition of CPT1 gene)
SEQ ID NO: 21: Primer 3 (for acquisition of CPT2 gene)
SEQ ID NO: 22: Primer 4 (for acquisition of CPT2 gene)
SEQ ID NO: 23: Primer 5 (for acquisition of CPT6 gene)
SEQ ID NO: 24: Primer 6 (for acquisition of CPT6 gene)
SEQ ID NO: 25: Primer 7 (for acquisition of CPT7 gene)
SEQ ID NO: 26: Primer 8 (for acquisition of CPT7 gene)
SEQ ID NO: 27: Primer 9 (for acquisition of CPTL gene)
SEQ ID NO: 28: Primer 10 (for acquisition of CPTL gene)
SEQ ID NO: 29: Primer 11 (for acquisition of REF1 gene)
SEQ ID NO: 30: Primer 12 (for acquisition of REF1 gene)
SEQ ID NO: 31: Primer 13 (for acquisition of REF2 gene)
SEQ ID NO: 32: Primer 14 (for acquisition of REF2 gene)
SEQ ID NO: 33: Primer 15 (for acquisition of REF8 gene)
SEQ ID NO: 34: Primer 16 (for acquisition of REF8 gene)
SEQ ID NO: 35: Primer 17 (for acquisition of SRPP1 gene)
SEQ ID NO: 36: Primer 18 (for acquisition of SRPP1 gene)

## Claims

1. A lipid membrane structure comprising a protein composition that exhibits an isoprene polymerization activity and a phospholipid, the protein composition comprising a protein(s) (B) and either one of proteins (A-1) and (A-2):
(A-1) one or more protein(s) selected from a protein(s) comprising an amino acid sequence of SEQ ID NO: 6 and a protein(s) that comprises an amino acid sequence having 90% or more identity with the amino acid sequence of SEQ ID NO: 6 and exhibits the same activity as CPT6;
(A-2) one or more protein(s) selected from a protein(s) comprising an amino acid sequence of SEQ ID NO: 8 and a protein(s) that comprises an amino acid sequence having 90% or more identity with the amino acid sequence of SEQ ID NO: 8 and exhibits the same activity as CPT7; and
(B) one or more protein(s) selected from a protein(s) comprising an amino acid sequence of SEQ ID NO: 10 and a protein(s) that comprises an amino acid sequence having 90% or more identity with the amino acid sequence of SEQ ID NO: 10 and exhibits the same activity as CPTL; and
the composition preferably fulfills at least one of the followings:
the protein (A-1) encoded by one or more polynucleotide(s) (a-1) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
the protein (A-2) encoded by one or more polynucleotide(s) (a-2) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
the protein (B) encoded by one or more polynucleotide(s) (b) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein exhibiting the same activity as CPTL; and
wherein the structure is a lipid bilayered membrane structure, or a proteoliposome.

2. The lipid membrane structure according to claim 1, wherein the composition further comprises an REF family protein, an SRPP family protein, and a CPT family protein other than CPT6, 7 and CPTL.

3. The lipid membrane structure according to claim 1, wherein the composition further comprises at least one of the followings:
(C) one or more protein(s) selected from a protein(s) comprising an amino acid sequence of SEQ ID NO: 2 and a protein(s) that comprises an amino acid sequence having 90% or more identity with the amino acid sequence of SEQ ID NO: 2 and exhibits the same activity as CPT1;
(D) one or more protein(s) selected from a protein(s) comprising an amino acid sequence of SEQ ID NO: 4 and a protein(s) that comprises an amino acid sequence having 90% or more identity with the amino acid sequence of SEQ ID NO: 4 and exhibits the same activity as CPT2;
(E) one or more protein(s) selected from a protein(s) comprising an amino acid sequence of SEQ ID NO: 12 and a protein(s) that comprises an amino acid sequence having 90% or more identity with the amino acid sequence of SEQ ID NO: 12 and exhibits the same activity as REF1;
(F) one or more protein(s) selected from a protein(s) comprising an amino acid sequence of SEQ ID NO: 14 and a protein(s) that comprises an amino acid sequence having 90% or more identity with the amino acid sequence of SEQ ID NO: 14 and exhibits the same activity as REF2;
(G) one or more protein(s) selected from a protein(s) comprising an amino acid sequence of SEQ ID NO: 16 and a protein(s) that comprises an amino acid sequence having 90% or more identity with the amino acid sequence of SEQ ID NO: 16 and exhibits the same activity as REF8; and
(H) one or more protein(s) selected from a protein(s) comprising an amino acid sequence of SEQ ID NO: 18 and a protein(s) that comprises an amino acid sequence having 90% or more identity with the amino acid sequence of SEQ ID NO: 18 and exhibits the same activity as SRPP1; and
the composition preferably fulfills at least one of the followings:
the protein (C) encoded by one or more polynucleotide(s) (c) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
the protein (D) encoded by one or more polynucleotide(s) (d) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
the protein (E) encoded by one or more polynucleotide(s) (e) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
the protein (F) encoded by one or more polynucleotide(s) (f) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
the protein (G) encoded by one or more polynucleotide(s) (g) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
the protein (H) encoded by one or more polynucleotide(s) (h) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.

4. The lipid membrane structure according to any one of claims 1 to 3, wherein at least one of the proteins (A) through (H) is a protein derived from a rubber tree.

5. A method for producing the lipid membrane structure according to any one of claims 1 to 4, the method comprising expressing the polynucleotide encoding the protein constituting the composition comprised by the structure according to any one of claims 1 to 4 in a cell-free protein production system in the presence of a raw material containing a phospholipid,
wherein the polynucleotide encoding the protein constituting the composition comprised by the structure according to any one of claims 1 to 4 preferably comprises (b) and either one of (a-1) and (a-2):
(a-1) one or more polynucleotide(s) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
(a-2) one or more polynucleotide(s) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
(b) one or more polynucleotide(s) selected from a polynucleotide(s) comprising a nucleic acid sequence of SEQ 6 ID NO: 9 and a polynucleotide(s) that comprises a nucleic acid sequence having 90% or more identity with the nucleic acid sequence of SEQ ID NO: 9 and encodes a protein exhibiting the same activity as CPTL.

6. The method for producing the lipid membrane structure according to claim 5, wherein the polynucleotide is the polynucleotide encoding the protein constituting the composition according to claim 2 or 3, and comprises at least one of the followings:
(c) one or more polynucleotide(s) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
(d) one or more polynucleotide(s) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
(e) one or more polynucleotide(s) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
(f) one or more polynucleotide(s) selected from a polynucleotide(s) comprising a nucleic acid sequence of SEQ ID NO: 13 and a polynucleotide(s) that comprises a nucleic acid sequence having 90% or more identity with the nucleic acid sequence of SEQ ID NO: 13 and encodes a protein exhibiting the same activity as REF2;
(g) one or more polynucleotide(s) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
(h) one or more polynucleotide(s) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.

7. The method for producing the lipid membrane structure according to claim 5 or 6, wherein the raw material containing the phospholipid contains a liposome, and wherein the lipid membrane structure is a proteoliposome.

8. A cell comprising the structure according to any one of claims 1 to 4,
wherein the cell preferably comprises an expression unit containing a polynucleotide that encodes each of the proteins in the protein composition comprised by the structure of any one of claims 1 to 4.

9. The cell according to claim 8, wherein the polynucleotide comprises (b) and either one of (a-1) and (a-2):
(a-1) one or more polynucleotide(s) selected from a polynucleotide(s) comprising a nucleic acid sequence of SEQ ID NO: 5 and a polynucleotide(s) that comprises a nucleic acid sequence having 90% or more identity with the nucleic acid sequence of SEQ ID NO: 5 and encodes a protein exhibiting the same activity as CPT6;
(a-2) one or more polynucleotide(s) selected from a polynucleotide(s) comprising a nucleic acid sequence of SEQ ID NO: 7 and a polynucleotide(s) that comprises a nucleic acid sequence having 90% or more identity with the nucleic acid sequence of SEQ ID NO: 7 and encodes a protein exhibiting the same activity as CPT7; and
(b) one or more polynucleotide(s) selected from a polynucleotide(s) comprising a nucleic acid sequence of SEQ ID NO: 9 and a polynucleotide(s) that comprises a nucleic acid sequence having 90% or more identity with the nucleic acid sequence of SEQ ID NO: 9 and encodes a protein 15 exhibiting the same activity as CPTL.

10. The cell according to claim 8 or 9, wherein the polynucleotide is the polynucleotide encoding the protein constituting the composition comprised by the structure according to claim 2 or 3, and further comprises at least one of the followings:
(c) one or more polynucleotide(s) selected from a polynucleotide(s) comprising a nucleic acid sequence of SEQ ID NO: 1 and a polynucleotide(s) that comprises a nucleic acid sequence having 90% or more identity with the nucleic acid sequence of SEQ ID NO: 1 and encodes a protein exhibiting the same activity as CPT1;
(d) one or more polynucleotide(s) selected from a polynucleotide(s) comprising a nucleic acid sequence of SEQ ID NO: 3 and a polynucleotide(s) that comprises a nucleic acid sequence having 90% or more identity with the nucleic acid sequence of SEQ ID NO: 3 and encodes a protein exhibiting the same activity as CPT2;
(e) one or more polynucleotide(s) selected from a polynucleotide(s) comprising a nucleic acid sequence of SEQ ID NO: 11 and a polynucleotide(s) that comprises a nucleic acid sequence having 90% or more identity with the nucleic acid sequence of SEQ ID NO: 11 and encodes a protein exhibiting the same activity as REF1;
(f) one or more polynucleotide(s) selected from a polynucleotide(s) comprising a nucleic acid sequence of SEQ ID NO: 13 and a polynucleotide(s) that comprises a nucleic acid sequence having 90% or more identity with the nucleic 10 acid sequence of SEQ ID NO: 13 and encodes a protein exhibiting the same activity as REF2;
(g) one or more polynucleotide(s) selected from a polynucleotide(s) comprising a nucleic acid sequence of SEQ ID NO: 15 and a polynucleotide(s) that comprises a nucleic acid sequence having 90% or more identity with the nucleic acid sequence of SEQ ID NO: 15 and encodes a protein exhibiting the same activity as REF8; and
(h) one or more polynucleotide(s) selected from a polynucleotide(s) comprising a nucleic acid sequence of SEQ ID NO: 17 and a polynucleotide(s) that comprises a nucleic acid sequence having 90% or more identity with the nucleic acid sequence of SEQ ID NO: 17 and encodes a protein exhibiting the same activity as SRPP1.

11. The cell according to any one of claims 8 to 10, wherein the expression unit is a heterologous expression unit.

12. The cell according to claim 8, comprising the lipid membrane structure according to claim 5,
wherein the structure is a proteoliposome.

13. A method for producing an isoprene polymer compound, the method comprising performing an isoprene polymerization reaction using at least one selected from the structure according to any one of claim 1 to 4, and the cell according to any one of claims 8 to 12; and
wherein the isoprene polymerization reaction is preferably performed using a low molecular weight allyl compound as a substrate.

14. A method for producing a rubber, the method comprising producing the rubber using an isoprene polymer compound, wherein said method further comprises a step of performing an isoprene polymerization reaction according to claim 13.

## Patentansprüche

1. Lipidmembranstruktur, umfassend eine Proteinzusammensetzung, die eine Isoprenpolymerisationsaktivität aufweist, und ein Phospholipid, wobei die Proteinzusammensetzung ein oder mehrere Proteine (B) und entweder eines der Proteine (A-1) und (A-2) umfasst:
(A-1) ein oder mehrere Proteine, ausgewählt aus Proteinen, die eine Aminosäuresequenz von SEQ ID NO: 6 umfassen, und Proteinen, die eine Aminosäuresequenz umfassen, die 90% oder mehr Identität mit der Aminosäuresequenz von SEQ ID NO: 6 aufweist und dieselbe Aktivität wie CPT6 aufweist;
(A-2) ein oder mehrere Proteine, ausgewählt aus Proteinen, die eine Aminosäuresequenz von SEQ ID NO: 8 umfassen, und Proteinen, die eine Aminosäuresequenz umfassen, die 90% oder mehr Identität mit der Aminosäuresequenz von SEQ ID NO: 8 aufweist und dieselbe Aktivität wie CPT7 aufweist; und
(B) ein oder mehrere Proteine, ausgewählt aus Proteinen, die eine Aminosäuresequenz von SEQ ID NO: 10 umfassen, und Proteinen, die eine Aminosäuresequenz umfassen, die 90% oder mehr Identität mit der Aminosäuresequenz von SEQ ID NO: 10 aufweist und dieselbe Aktivität wie CPTL aufweist; und
wobei die Zusammensetzung bevorzugt mindestens eines der Folgenden erfüllt:
das Protein (A-1), das von einem oder mehreren Polynukleotiden (a-1) kodiert wird, ausgewählt aus Polynukleotiden, die eine Nukleinsäuresequenz von SEQ ID NO: 5 umfassen, und Polynukleotiden, die eine Nukleinsäuresequenz umfassen, die 90% oder mehr Identität mit der Nukleinsäuresequenz von SEQ ID NO: 5 aufweist, und ein Protein kodiert, das dieselbe Aktivität wie CPT6 aufweist;
das Protein (A-2), das von einem oder mehreren Polynukleotiden (a-2) kodiert wird, ausgewählt aus Polynukleotiden, die eine Nukleinsäuresequenz von SEQ ID NO: 7 umfassen, und Polynukleotiden, die eine Nukleinsäuresequenz umfassen, die 90% oder mehr Identität mit der Nukleinsäuresequenz von SEQ ID NO: 7 aufweist, und ein Protein kodiert, das dieselbe Aktivität wie CPT7 aufweist; und
das Protein (B), das von einem oder mehreren Polynukleotiden (b) kodiert wird, ausgewählt aus Polynukleotiden, die eine Nukleinsäuresequenz von SEQ ID NO: 9 umfassen, und Polynukleotiden, die eine Nukleinsäuresequenz umfassen, die 90% oder mehr Identität mit der Nukleinsäuresequenz von SEQ ID NO: 9 aufweist, und ein Protein kodiert, das dieselbe Aktivität wie CPTL aufweist; und
wobei die Struktur eine Lipid-Doppelschichtmembranstruktur oder ein Proteoliposom ist.

2. Lipidmembranstruktur nach Anspruch 1, wobei die Zusammensetzung weiter ein Protein der REF-Familie, ein Protein der SRPP-Familie und ein Protein der CPT-Familie außer CPT6, 7 und CPTL umfasst.

3. Lipidmembranstruktur nach Anspruch 1, wobei die Zusammensetzung weiter mindestens eines der Folgenden umfasst:
(C) ein oder mehrere Proteine, ausgewählt aus Proteinen, die eine Aminosäuresequenz von SEQ ID NO: 2 umfassen, und Proteinen, die eine Aminosäuresequenz umfassen, die 90% oder mehr Identität mit der Aminosäuresequenz von SEQ ID NO: 2 aufweist und dieselbe Aktivität wie CPT1 aufweist;
(D) ein oder mehrere Proteine, ausgewählt aus Proteinen, die eine Aminosäuresequenz von SEQ ID NO: 4 umfassen, und Proteinen, die eine Aminosäuresequenz umfassen, die 90% oder mehr Identität mit der Aminosäuresequenz von SEQ ID NO: 4 aufweist und dieselbe Aktivität wie CPT2 aufweist;
(E) ein oder mehrere Proteine, ausgewählt aus Proteinen, die eine Aminosäuresequenz von SEQ ID NO: 12 umfassen, und Proteinen, die eine Aminosäuresequenz umfassen, die 90% oder mehr Identität mit der Aminosäuresequenz von SEQ ID NO: 12 aufweist und dieselbe Aktivität wie REF1 aufweist;
(F) ein oder mehrere Proteine, ausgewählt aus Proteinen, die eine Aminosäuresequenz von SEQ ID NO: 14 umfassen, und Proteinen, die eine Aminosäuresequenz umfassen, die 90% oder mehr Identität mit der Aminosäuresequenz von SEQ ID NO: 14 aufweist und dieselbe Aktivität wie REF2 aufweist;
(G) ein oder mehrere Proteine, ausgewählt aus Proteinen, die eine Aminosäuresequenz von SEQ ID NO: 16 umfassen, und Proteinen, die eine Aminosäuresequenz umfassen, die 90% oder mehr Identität mit der Aminosäuresequenz von SEQ ID NO: 16 aufweist und dieselbe Aktivität wie REF8 aufweist; und
(H) ein oder mehrere Proteine, ausgewählt aus Proteinen, die eine Aminosäuresequenz von SEQ ID NO: 18 umfassen, und Proteinen, die eine Aminosäuresequenz umfassen, die 90% oder mehr Identität mit der Aminosäuresequenz von SEQ ID NO: 18 aufweist und dieselbe Aktivität wie SRPP1 aufweist; und
wobei die Zusammensetzung bevorzugt mindestens eines der Folgenden erfüllt:
das Protein (C), das von einem oder mehreren Polynukleotiden (c) kodiert wird, ausgewählt aus Polynukleotiden, die eine Nukleinsäuresequenz von SEQ ID NO: 1 umfassen, und Polynukleotiden, die eine Nukleinsäuresequenz umfassen, die 90% oder mehr Identität mit der Nukleinsäuresequenz von SEQ ID NO: 1 aufweist, und ein Protein kodiert, das dieselbe Aktivität wie CPT1 aufweist;
das Protein (D), das von einem oder mehreren Polynukleotiden (d) kodiert wird, ausgewählt aus Polynukleotiden, die eine Nukleinsäuresequenz von SEQ ID NO: 3 umfassen, und Polynukleotiden, die eine Nukleinsäuresequenz umfassen, die 90% oder mehr Identität mit der Nukleinsäuresequenz von SEQ ID NO: 3 aufweist, und ein Protein kodiert, das dieselbe Aktivität wie CPT2 aufweist;
das Protein (E), das von einem oder mehreren Polynukleotiden (e) kodiert wird, ausgewählt aus Polynukleotiden, die eine Nukleinsäuresequenz von SEQ ID NO: 11 umfassen, und Polynukleotiden, die eine Nukleinsäuresequenz umfassen, die 90% oder mehr Identität mit der Nukleinsäuresequenz von SEQ ID NO: 11 aufweist, und ein Protein kodiert, das dieselbe Aktivität wie REF1 aufweist;
das Protein (F), das von einem oder mehreren Polynukleotiden (f) kodiert wird, ausgewählt aus Polynukleotiden, die eine Nukleinsäuresequenz von SEQ ID NO: 13 umfassen, und Polynukleotiden, die eine Nukleinsäuresequenz umfassen, die 90% oder mehr Identität mit der Nukleinsäuresequenz von SEQ ID NO: 13 aufweist, und ein Protein kodiert, das dieselbe Aktivität wie REF2 aufweist;
das Protein (G), das von einem oder mehreren Polynukleotiden (g) kodiert wird, ausgewählt aus Polynukleotiden, die eine Nukleinsäuresequenz von SEQ ID NO: 15 umfassen, und Polynukleotiden, die eine Nukleinsäuresequenz umfassen, die 90% oder mehr Identität mit der Nukleinsäuresequenz von SEQ ID NO: 15 aufweist, und ein Protein kodiert, das dieselbe Aktivität wie REF8 aufweist; und
das Protein (H), das von einem oder mehreren Polynukleotiden (h) kodiert wird, ausgewählt aus Polynukleotiden, die eine Nukleinsäuresequenz von SEQ ID NO: 17 umfassen, und Polynukleotiden, die eine Nukleinsäuresequenz umfassen, die 90% oder mehr Identität mit der Nukleinsäuresequenz von SEQ ID NO: 17 aufweist, und ein Protein kodiert, das dieselbe Aktivität wie SRPP1 aufweist.

4. Lipidmembranstruktur nach einem der Ansprüche 1 bis 3, wobei mindestens eines der Proteine (A) bis (H) ein Protein ist, das von einem Kautschukbaum abgeleitet ist.

5. Erzeugungsverfahren zur Erzeugung der Lipidmembranstruktur nach einem der Ansprüche 1 bis 4, das Verfahren umfassend das Exprimieren des Polynukleotids, das das Protein kodiert, das die von der Struktur nach einem der Ansprüche 1 bis 4 umfasste Zusammensetzung konstituiert, in einem zellfreien Proteinsynthesesystem in Gegenwart eines phospholipidhaltigen Ausgangsmaterials,
wobei das Polynukleotid, das das Protein kodiert, das die von der Struktur nach einem der Ansprüche 1 bis 4 umfasste Zusammensetzung konstituiert, bevorzugt (b) und entweder eines von (a-1) und (a-2) umfasst:
(a-1) ein oder mehrere Polynukleotide, ausgewählt aus Polynukleotiden, die eine Nukleinsäuresequenz von SEQ ID NO: 5 umfassen, und Polynukleotiden, die eine Nukleinsäuresequenz umfassen, die 90% oder mehr Identität mit der Nukleinsäuresequenz von SEQ ID NO: 5 aufweist, und ein Protein kodiert, das dieselbe Aktivität wie CPT6 aufweist;
(a-2) ein oder mehrere Polynukleotide, ausgewählt aus Polynukleotiden, die eine Nukleinsäuresequenz von SEQ ID NO: 7 umfassen, und Polynukleotiden, die eine Nukleinsäuresequenz umfassen, die 90% oder mehr Identität mit der Nukleinsäuresequenz von SEQ ID NO: 7 aufweist, und ein Protein kodiert, das dieselbe Aktivität wie CPT7 aufweist; und
(b) ein oder mehrere Polynukleotide, ausgewählt aus Polynukleotiden, die eine Nukleinsäuresequenz von SEQ ID NO: 9 umfassen, und Polynukleotiden, die eine Nukleinsäuresequenz umfassen, die 90% oder mehr Identität mit der Nukleinsäuresequenz von SEQ ID NO: 9 aufweist, und ein Protein kodiert, das dieselbe Aktivität wie CPTL aufweist.

6. Erzeugungsverfahren zur Erzeugung der Lipidmembranstruktur nach Anspruch 5, wobei das Polynukleotid das Polynukleotid ist, das die Zusammensetzung nach Anspruch 2 oder 3 konstituierende Protein kodiert, und mindestens eines der Folgenden umfasst:
(c) ein oder mehrere Polynukleotide, ausgewählt aus Polynukleotiden, die eine Nukleinsäuresequenz von SEQ ID NO: 1 umfassen, und Polynukleotiden, die eine Nukleinsäuresequenz umfassen, die 90% oder mehr Identität mit der Nukleinsäuresequenz von SEQ ID NO: 1 aufweist, und ein Protein kodiert, das dieselbe Aktivität wie CPT1 aufweist;
(d) ein oder mehrere Polynukleotide, ausgewählt aus Polynukleotiden, die eine Nukleinsäuresequenz von SEQ ID NO: 3 umfassen, und Polynukleotiden, die eine Nukleinsäuresequenz umfassen, die 90% oder mehr Identität mit der Nukleinsäuresequenz von SEQ ID NO: 3 aufweist, und ein Protein kodiert, das dieselbe Aktivität wie CPT2 aufweist;
(e) ein oder mehrere Polynukleotide, ausgewählt aus Polynukleotiden, die eine Nukleinsäuresequenz von SEQ ID NO: 11 umfassen, und Polynukleotiden, die eine Nukleinsäuresequenz umfassen, die 90% oder mehr Identität mit der Nukleinsäuresequenz von SEQ ID NO: 11 aufweist, und ein Protein kodiert, das dieselbe Aktivität wie REF1 aufweist;
(f) ein oder mehrere Polynukleotide, ausgewählt aus Polynukleotiden, die eine Nukleinsäuresequenz von SEQ ID NO: 13 umfassen, und Polynukleotiden, die eine Nukleinsäuresequenz umfassen, die 90% oder mehr Identität mit der Nukleinsäuresequenz von SEQ ID NO: 13 aufweist, und ein Protein kodiert, das dieselbe Aktivität wie REF2 aufweist;
(g) ein oder mehrere Polynukleotide, ausgewählt aus Polynukleotiden, die eine Nukleinsäuresequenz von SEQ ID NO: 15 umfassen, und Polynukleotiden, die eine Nukleinsäuresequenz umfassen, die 90% oder mehr Identität mit der Nukleinsäuresequenz von SEQ ID NO: 15 aufweist, und ein Protein kodiert, das dieselbe Aktivität wie REF8 aufweist; und
(h) ein oder mehrere Polynukleotide, ausgewählt aus Polynukleotiden, die eine Nukleinsäuresequenz von SEQ ID NO: 17 umfassen, und Polynukleotiden, die eine Nukleinsäuresequenz umfassen, die 90% oder mehr Identität mit der Nukleinsäuresequenz von SEQ ID NO: 17 aufweist, und ein Protein kodiert, das dieselbe Aktivität wie SRPP1 aufweist.

7. Erzeugungsverfahren zur Erzeugung der Lipidmembranstruktur nach Anspruch 5 oder 6, wobei das phospholipidhaltige Ausgangsmaterial ein Liposom enthält, und wobei die Lipidmembranstruktur ein Proteoliposom ist.

8. Zelle, umfassend die Struktur nach einem der Ansprüche 1 bis 4,
wobei die Zelle bevorzugt eine Expressionseinheit umfasst, die ein Polynukleotid enthält, das jedes der Proteine in der von der Struktur eines der Ansprüche 1 bis 4 umfassten Proteinzusammensetzung kodiert.

9. Zelle nach Anspruch 8, wobei das Polynukleotid (b) und entweder eines von (a-1) und (a-2) umfasst:
(a-1) ein oder mehrere Polynukleotide, ausgewählt aus Polynukleotiden, die eine Nukleinsäuresequenz von SEQ ID NO: 5 umfassen, und Polynukleotiden, die eine Nukleinsäuresequenz umfassen, die 90% oder mehr Identität mit der Nukleinsäuresequenz von SEQ ID NO: 5 aufweist, und ein Protein kodiert, das dieselbe Aktivität wie CPT6 aufweist;
(a-2) ein oder mehrere Polynukleotide, ausgewählt aus Polynukleotiden, die eine Nukleinsäuresequenz von SEQ ID NO: 7 umfassen, und Polynukleotiden, die eine Nukleinsäuresequenz umfassen, die 90% oder mehr Identität mit der Nukleinsäuresequenz von SEQ ID NO: 7 aufweist, und ein Protein kodiert, das dieselbe Aktivität wie CPT7 aufweist; und
(b) ein oder mehrere Polynukleotide, ausgewählt aus Polynukleotiden, die eine Nukleinsäuresequenz von SEQ ID NO: 9 umfassen, und Polynukleotiden, die eine Nukleinsäuresequenz umfassen, die 90% oder mehr Identität mit der Nukleinsäuresequenz von SEQ ID NO: 9 aufweist, und ein Protein 15 kodiert, das dieselbe Aktivität wie CPTL aufweist.

10. Zelle nach Anspruch 8 oder 9, wobei das Polynukleotid das Polynukleotid ist, das das Protein kodiert, das die von der Struktur nach Anspruch 2 oder 3 umfasste Zusammensetzung konstituiert, und weiter mindestens eines der Folgenden umfasst:
(c) ein oder mehrere Polynukleotide, ausgewählt aus Polynukleotiden, die eine Nukleinsäuresequenz von SEQ ID NO: 1 umfassen, und Polynukleotiden, die eine Nukleinsäuresequenz umfassen, die 90% oder mehr Identität mit der Nukleinsäuresequenz von SEQ ID NO: 1 aufweist, und ein Protein kodiert, das dieselbe Aktivität wie CPT1 aufweist;
(d) ein oder mehrere Polynukleotide, ausgewählt aus Polynukleotiden, die eine Nukleinsäuresequenz von SEQ ID NO: 3 umfassen, und Polynukleotiden, die eine Nukleinsäuresequenz umfassen, die 90% oder mehr Identität mit der Nukleinsäuresequenz von SEQ ID NO: 3 aufweist, und ein Protein kodiert, das dieselbe Aktivität wie CPT2 aufweist;
(e) ein oder mehrere Polynukleotide, ausgewählt aus Polynukleotiden, die eine Nukleinsäuresequenz von SEQ ID NO: 11 umfassen, und Polynukleotiden, die eine Nukleinsäuresequenz umfassen, die 90% oder mehr Identität mit der Nukleinsäuresequenz von SEQ ID NO: 11 aufweist, und ein Protein kodiert, das dieselbe Aktivität wie REF1 aufweist;
(f) ein oder mehrere Polynukleotide, ausgewählt aus Polynukleotiden, die eine Nukleinsäuresequenz von SEQ ID NO: 13 umfassen, und Polynukleotiden, die eine Nukleinsäuresequenz umfassen, die 90% oder mehr Identität mit der Nukleinsäure 10 Sequenz von SEQ ID NO: 13 aufweist, und ein Protein kodiert, das dieselbe Aktivität wie REF2 aufweist;
(g) ein oder mehrere Polynukleotide, ausgewählt aus Polynukleotiden, die eine Nukleinsäuresequenz von SEQ ID NO: 15 umfassen, und Polynukleotiden, die eine Nukleinsäuresequenz umfassen, die 90% oder mehr Identität mit der Nukleinsäuresequenz von SEQ ID NO: 15 aufweist, und ein Protein kodiert, das dieselbe Aktivität wie REF8 aufweist; und
(h) ein oder mehrere Polynukleotide, ausgewählt aus Polynukleotiden, die eine Nukleinsäuresequenz von SEQ ID NO: 17 umfassen, und Polynukleotiden, die eine Nukleinsäuresequenz umfassen, die 90% oder mehr Identität mit der Nukleinsäuresequenz von SEQ ID NO: 17 aufweist, und ein Protein kodiert, das dieselbe Aktivität wie SRPP1 aufweist.

11. Zelle nach einem der Ansprüche 8 bis 10, wobei die Expressionseinheit eine heterologe Expressionseinheit ist.

12. Zelle nach Anspruch 8, umfassend die Lipidmembranstruktur nach Anspruch 5, wobei die Struktur ein Proteoliposom ist.

13. Erzeugungsverfahren zur Erzeugung einer Isoprenpolymerverbindung, das Verfahren umfassend das Durchführen einer Isoprenpolymerisationsreaktion unter Verwendung von mindestens einem ausgewählt aus der Struktur nach einem der Ansprüche 1 bis 4, und der Zelle nach einem der Ansprüche 8 bis 12; und,
wobei die Isoprenpolymerisationsreaktion bevorzugt unter Verwendung einer niedermolekularen Allylverbindung als Substrat durchgeführt wird.

14. Erzeugungsverfahren zur Erzeugung eines Kautschuks, das Verfahren umfassend das Erzeugen des Kautschuks unter Verwendung einer Isoprenpolymerverbindung, wobei das Verfahren weiter einen Schritt des Durchführens einer Isoprenpolymerisationsreaktion nach Anspruch 13 umfasst.

## Revendications

1. Structure de membrane lipidique comprenant une composition protéique qui présente une activité de polymérisation de l'isoprène et un phospholipide, la composition protéique comprenant une ou plusieurs protéine(s) (B) et l'une quelconque des protéines (A-1) et (A-2) :
(A-1) une ou plusieurs protéine(s) sélectionnée(s) parmi une ou plusieurs protéine(s) comprenant une séquence d'acides aminés de SEQ ID N° 6 et une ou plusieurs protéine(s) qui comprend une séquence d'acides aminés présentant une identité de 90 % ou plus avec la séquence d'acides aminés de SEQ ID N° 6 et qui présente la même activité que CPT6 ;
(A-2) une ou plusieurs protéine(s) sélectionnée(s) parmi une ou plusieurs protéine(s) comprenant une séquence d'acides aminés de SEQ ID N° 8 et une ou plusieurs protéine(s) qui comprend une séquence d'acides aminés présentant une identité de 90 % ou plus avec la séquence d'acides aminés de SEQ ID N° 8 et qui présente la même activité que CPT7 ; et
(B) une ou plusieurs protéine(s) sélectionnée(s) parmi une ou plusieurs protéine(s) comprenant une séquence d'acides aminés de SEQ ID N° 10 et une ou plusieurs protéine(s) qui comprend une séquence d'acides aminés présentant une identité de 90 % ou plus avec la séquence d'acides aminés de SEQ ID N° 10 et qui présente la même activité que CPTL ; et
la composition répondant de préférence à au moins un parmi ce qui suit :
la protéine (A-1) est codée par un ou plusieurs polynucléotide(s) (a-1) sélectionné(s) parmi un ou plusieurs polynucléotide(s) comprenant une séquence d'acide nucléique de SEQ ID N° 5 et un ou plusieurs polynucléotide(s) qui comprend une séquence d'acide nucléique présentant une identité de 90 % ou plus avec la séquence d'acide nucléique de SEQ ID N° 5 et qui code une protéine présentant la même activité que CPT6 ;
la protéine (A-2) est codée par un ou plusieurs polynucléotide(s) (a-2) sélectionné(s) parmi un ou plusieurs polynucléotide(s) comprenant une séquence d'acide nucléique de SEQ ID N° 7 et un ou plusieurs polynucléotide(s) qui comprend une séquence d'acide nucléique présentant une identité de 90 % ou plus avec la séquence d'acide nucléique de SEQ ID N° 7 et qui code une protéine présentant la même activité que CPT7 ; et
la protéine (B) est codée par un ou plusieurs polynucléotide(s) (b) sélectionné(s) parmi un ou plusieurs polynucléotide(s) comprenant une séquence d'acide nucléique de SEQ ID N° 9 et un ou plusieurs polynucléotide(s) qui comprend une séquence d'acide nucléique présentant une identité de 90 % ou plus avec la séquence d'acide nucléique de SEQ ID N° 9 et qui code une protéine présentant la même activité que CPTL ; et
dans laquelle la structure est une structure de membrane à bicouche lipidique, ou un protéoliposome.

2. Structure de membrane lipidique selon la revendication 1, dans laquelle la composition comprend en outre une protéine de la famille REF, une protéine de la famille SRPP, et une protéine de la famille CPT autre que CPT6, 7 et CPTL.

3. Structure de membrane lipidique selon la revendication 1, dans laquelle la composition comprend en outre au moins un parmi ce qui suit :
(C) une ou plusieurs protéine(s) sélectionnée(s) parmi une ou plusieurs protéine(s) comprenant une séquence d'acides aminés de SEQ ID N° 2 et une ou plusieurs protéine(s) qui comprend une séquence d'acides aminés présentant une identité de 90 % ou plus avec la séquence d'acides aminés de SEQ ID N° 2 et qui présente la même activité que CPT1 ;
(D) une ou plusieurs protéine(s) sélectionnée(s) parmi une ou plusieurs protéine(s) comprenant une séquence d'acides aminés de SEQ ID N° 4 et une ou plusieurs protéine(s) qui comprend une séquence d'acides aminés présentant une identité de 90 % ou plus avec la séquence d'acides aminés de SEQ ID N° 4 et qui présente la même activité que CPT2 ;
(E) une ou plusieurs protéine(s) sélectionnée(s) parmi une ou plusieurs protéine(s) comprenant une séquence d'acides aminés de SEQ ID N° 12 et une ou plusieurs protéine(s) qui comprend une séquence d'acides aminés présentant une identité de 90 % ou plus avec la séquence d'acides aminés de SEQ ID N° 12 et qui présente la même activité que REF1 ;
(F) une ou plusieurs protéine(s) sélectionnée(s) parmi une ou plusieurs protéine(s) comprenant une séquence d'acides aminés de SEQ ID N° 14 et une ou plusieurs protéine(s) qui comprend une séquence d'acides aminés présentant une identité de 90 % ou plus avec la séquence d'acides aminés de SEQ ID N° 14 et qui présente la même activité que REF2 ;
(G) une ou plusieurs protéine(s) sélectionnée(s) parmi une ou plusieurs protéine(s) comprenant une séquence d'acides aminés de SEQ ID N° 16 et une ou plusieurs protéine(s) qui comprend une séquence d'acides aminés présentant une identité de 90 % ou plus avec la séquence d'acides aminés de SEQ ID N° 16 et qui présente la même activité que REF8 ; et
(H) une ou plusieurs protéine(s) sélectionnée(s) parmi une ou plusieurs protéine(s) comprenant une séquence d'acides aminés de SEQ ID N° 18 et une ou plusieurs protéine(s) qui comprend une séquence d'acides aminés présentant une identité de 90 % ou plus avec la séquence d'acides aminés de SEQ ID N° 18 et qui présente la même activité que SRPP1 ; et
la composition répond de préférence à au moins un parmi ce qui suit :
la protéine (C) est codée par un ou plusieurs polynucléotide(s) (c) sélectionné(s) parmi un ou plusieurs polynucléotide(s) comprenant une séquence d'acide nucléique de SEQ ID N° 1 et un ou plusieurs polynucléotide(s) qui comprend une séquence d'acide nucléique présentant une identité de 90 % ou plus avec la séquence d'acide nucléique de SEQ ID N° 1 et qui code une protéine présentant la même activité que CPT1 ;
la protéine (D) est codée par un ou plusieurs polynucléotide(s) (d) sélectionné(s) parmi un ou plusieurs polynucléotide(s) comprenant une séquence d'acide nucléique de SEQ ID N° 3 et un ou plusieurs polynucléotide(s) qui comprend une séquence d'acide nucléique présentant une identité de 90 % ou plus avec la séquence d'acide nucléique de SEQ ID N° 3 et qui code une protéine présentant la même activité que CPT2 ;
la protéine (E) est codée par un ou plusieurs polynucléotide(s) (e) sélectionné(s) parmi un ou plusieurs polynucléotide(s) comprenant une séquence d'acide nucléique de SEQ ID N° 11 et un ou plusieurs polynucléotide(s) qui comprend une séquence d'acide nucléique présentant une identité de 90 % ou plus avec la séquence d'acide nucléique de SEQ ID N° 11 et qui code une protéine présentant la même activité que REF1 ;
la protéine (F) est codée par un ou plusieurs polynucléotide(s) (f) sélectionné(s) parmi un ou plusieurs polynucléotide(s) comprenant une séquence d'acide nucléique de SEQ ID N° 13 et un ou plusieurs polynucléotide(s) qui comprend une séquence d'acide nucléique présentant une identité de 90 % ou plus avec la séquence d'acide nucléique de SEQ ID N° 13 et qui code une protéine présentant la même activité que REF2 ;
la protéine (G) est codée par un ou plusieurs polynucléotide(s) (g) sélectionné(s) parmi un ou plusieurs polynucléotide(s) comprenant une séquence d'acide nucléique de SEQ ID N° 15 et un ou plusieurs polynucléotide(s) qui comprend une séquence d'acide nucléique présentant une identité de 90 % ou plus avec la séquence d'acide nucléique de SEQ ID N° 15 et qui code une protéine présentant la même activité que REF8 ; et
la protéine (H) est codée par un ou plusieurs polynucléotide(s) (h) sélectionné(s) parmi un ou plusieurs polynucléotide(s) comprenant une séquence d'acide nucléique de SEQ ID N° 17 et un ou plusieurs polynucléotide(s) qui comprend une séquence d'acide nucléique présentant une identité de 90 % ou plus avec la séquence d'acide nucléique de SEQ ID N° 17 et qui code une protéine présentant la même activité que SRPP1.

4. Structure de membrane lipidique selon l'une quelconque des revendications 1 à 3, dans laquelle au moins une des protéines (A) à (H) est une protéine dérivée d'un hévéa.

5. Procédé de production de la structure de membrane lipidique selon l'une quelconque des revendications 1 à 4, le procédé comprenant l'expression du polynucléotide codant la protéine constituant la composition comprise par la structure selon l'une quelconque des revendications 1 à 4 dans un système de synthèse protéique acellulaire en présence d'une matière première contenant un phospholipide, dans lequel le polynucléotide codant la protéine constituant la composition comprise par la structure selon l'une quelconque des revendications 1 à 4 comprend de préférence (b) et l'un quelconque de (a-1) et (a-2) :
(a-1) un ou plusieurs polynucléotide(s) sélectionné(s) parmi un ou plusieurs polynucléotide(s) comprenant une séquence d'acide nucléique de SEQ ID N° 5 et un ou plusieurs polynucléotide(s) qui comprend une séquence d'acide nucléique présentant une identité de 90 % ou plus avec la séquence d'acide nucléique de SEQ ID N° 5 et qui code une protéine présentant la même activité que CPT6 ;
(a-2) un ou plusieurs polynucléotide(s) sélectionné(s) parmi un ou plusieurs polynucléotide(s) comprenant une séquence d'acide nucléique de SEQ ID N° 7 et un ou plusieurs polynucléotide(s) qui comprend une séquence d'acide nucléique présentant une identité de 90 % ou plus avec la séquence d'acide nucléique de SEQ ID N° 7 et qui code une protéine présentant la même activité que CPT7 ; et
(b) un ou plusieurs polynucléotide(s) sélectionné(s) parmi un ou plusieurs polynucléotide(s) comprenant une séquence d'acide nucléique de SEQ ID N° 9 et un ou plusieurs polynucléotide(s) qui comprend une séquence d'acide nucléique présentant une identité de 90 % ou plus avec la séquence d'acide nucléique de SEQ ID N° 9 et qui code une protéine présentant la même activité que CPTL.

6. Procédé de production de la structure de membrane lipidique selon la revendication 5, dans lequel le polynucléotide est le polynucléotide codant la protéine constituant la composition selon la revendication 2 ou la revendication 3, et comprend au moins un parmi ce qui suit :
(c) un ou plusieurs polynucléotide(s) sélectionné(s) parmi un ou plusieurs polynucléotide(s) comprenant une séquence d'acide nucléique de SEQ ID N° 1 et un ou plusieurs polynucléotide(s) qui comprend une séquence d'acide nucléique présentant une identité de 90 % ou plus avec la séquence d'acide nucléique de SEQ ID N° 1 et qui code une protéine présentant la même activité que CPT1 ;
(d) un ou plusieurs polynucléotide(s) sélectionné(s) parmi un ou plusieurs polynucléotide(s) comprenant une séquence d'acide nucléique de SEQ ID N° 3 et un ou plusieurs polynucléotide(s) qui comprend une séquence d'acide nucléique présentant une identité de 90 % ou plus avec la séquence d'acide nucléique de SEQ ID N° 3 et qui code une protéine présentant la même activité que CPT2 ;
(e) un ou plusieurs polynucléotide(s) sélectionné(s) parmi un ou plusieurs polynucléotide(s) comprenant une séquence d'acide nucléique de SEQ ID N° 11 et un ou plusieurs polynucléotide(s) qui comprend une séquence d'acide nucléique présentant une identité de 90 % ou plus avec la séquence d'acide nucléique de SEQ ID N° 11 et qui code une protéine présentant la même activité que REF1 ;
(f) un ou plusieurs polynucléotide(s) sélectionné(s) parmi un ou plusieurs polynucléotide(s) comprenant une séquence d'acide nucléique de SEQ ID N° 13 et un ou plusieurs polynucléotide(s) qui comprend une séquence d'acide nucléique présentant une identité de 90 % ou plus avec la séquence d'acide nucléique de SEQ ID N° 13 et qui code une protéine présentant la même activité que REF2 ;
(g) un ou plusieurs polynucléotide(s) sélectionné(s) parmi un ou plusieurs polynucléotide(s) comprenant une séquence d'acide nucléique de SEQ ID N° 15 et un ou plusieurs polynucléotide(s) qui comprend une séquence d'acide nucléique présentant une identité de 90 % ou plus avec la séquence d'acide nucléique de SEQ ID N° 15 et qui code une protéine présentant la même activité que REF8 ; et
(h) un ou plusieurs polynucléotide(s) sélectionné(s) parmi un ou plusieurs polynucléotide(s) comprenant une séquence d'acide nucléique de SEQ ID N° 17 et un ou plusieurs polynucléotide(s) qui comprend une séquence d'acide nucléique présentant une identité de 90 % ou plus avec la séquence d'acide nucléique de SEQ ID N° 17 et qui code une protéine présentant la même activité que SRPP1.

7. Procédé de production de la structure de membrane lipidique selon la revendication 5 ou la revendication 6, dans lequel la matière première contenant le phospholipide contient un liposome, et dans lequel la structure de membrane lipidique est un protéoliposome.

8. Cellule comprenant la structure selon l'une quelconque des revendications 1 à 4,
dans laquelle la cellule comprend de préférence une unité d'expression contenant un polynucléotide qui code chacune des protéines dans la composition protéique comprise par la structure de l'une quelconque des revendications 1 à 4.

9. Cellule selon la revendication 8, dans laquelle le polynucléotide comprend (b) et un quelconque parmi (a-1) et (a-2) :
(a-1) un ou plusieurs polynucléotide(s) sélectionné(s) parmi un ou plusieurs polynucléotide(s) comprenant une séquence d'acide nucléique de SEQ ID N° 5 et un ou plusieurs polynucléotide(s) qui comprend une séquence d'acide nucléique présentant une identité de 90 % ou plus avec la séquence d'acide nucléique de SEQ ID N° 5 et qui code une protéine présentant la même activité que CPT6 ;
(a-2) un ou plusieurs polynucléotide(s) sélectionné(s) parmi un ou plusieurs polynucléotide(s) comprenant une séquence d'acide nucléique de SEQ ID N° 7 et un ou plusieurs polynucléotide(s) qui comprend une séquence d'acide nucléique présentant une identité de 90 % ou plus avec la séquence d'acide nucléique de SEQ ID N° 7 et qui code une protéine présentant la même activité que CPT7 ; et
(b) un ou plusieurs polynucléotide(s) sélectionné(s) parmi un ou plusieurs polynucléotide(s) comprenant une séquence d'acide nucléique de SEQ ID N° 9 et un ou plusieurs polynucléotide(s) qui comprend une séquence d'acide nucléique présentant une identité de 90 % ou plus avec la séquence d'acide nucléique de SEQ ID N° 9 et qui code une protéine présentant la même activité que CPTL.

10. Cellule selon la revendication 8 ou la revendication 9, dans laquelle le polynucléotide est le polynucléotide codant la protéine constituant la composition comprise par la structure selon la revendication 2 ou la revendication 3, et comprend en outre au moins un parmi ce qui suit :
(c) un ou plusieurs polynucléotide(s) sélectionné(s) parmi un ou plusieurs polynucléotide(s) comprenant une séquence d'acide nucléique de SEQ ID N° 1 et un ou plusieurs polynucléotide(s) qui comprend une séquence d'acide nucléique présentant une identité de 90 % ou plus avec la séquence d'acide nucléique de SEQ ID N° 1 et qui code une protéine présentant la même activité que CPT1 ;
(d) un ou plusieurs polynucléotide(s) sélectionné(s) parmi un ou plusieurs polynucléotide(s) comprenant une séquence d'acide nucléique de SEQ ID N° 3 et un ou plusieurs polynucléotide(s) qui comprend une séquence d'acide nucléique présentant une identité de 90 % ou plus avec la séquence d'acide nucléique de SEQ ID N° 3 et qui code une protéine présentant la même activité que CPT2 ;
(e) un ou plusieurs polynucléotide(s) sélectionné(s) parmi un ou plusieurs polynucléotide(s) comprenant une séquence d'acide nucléique de SEQ ID N° 11 et un ou plusieurs polynucléotide(s) qui comprend une séquence d'acide nucléique présentant une identité de 90 % ou plus avec la séquence d'acide nucléique de SEQ ID N° 11 et qui code une protéine présentant la même activité que REF1 ;
(f) un ou plusieurs polynucléotide(s) sélectionné(s) parmi un ou plusieurs polynucléotide(s) comprenant une séquence d'acide nucléique de SEQ ID N° 13 et un ou plusieurs polynucléotide(s) qui comprend une séquence d'acide nucléique présentant une identité de 90 % ou plus avec la séquence d'acide nucléique de SEQ ID N° 13 et qui code une protéine présentant la même activité que REF2 ;
(g) un ou plusieurs polynucléotide(s) sélectionné(s) parmi un ou plusieurs polynucléotide(s) comprenant une séquence d'acide nucléique de SEQ ID N° 15 et un ou plusieurs polynucléotide(s) qui comprend une séquence d'acide nucléique présentant une identité de 90 % ou plus avec la séquence d'acide nucléique de SEQ ID N° 15 et qui code une protéine présentant la même activité que REF8 ; et
(h) un ou plusieurs polynucléotide(s) sélectionné(s) parmi un ou plusieurs polynucléotide(s) comprenant une séquence d'acide nucléique de SEQ ID N° 17 et un ou plusieurs polynucléotide(s) qui comprend une séquence d'acide nucléique présentant une identité de 90 % ou plus avec la séquence d'acide nucléique de SEQ ID N° 17 et qui code une protéine présentant la même activité que SRPP1.

11. Cellule selon l'une quelconque des revendications 8 à 10, dans laquelle l'unité d'expression est une unité d'expression hétérologue.

12. Cellule selon la revendication 8, comprenant la structure de membrane lipidique selon la revendication 5, dans laquelle la structure est un protéoliposome.

13. Procédé de production d'un composé polymérique d'isoprène, le procédé comprenant l'exécution d'une réaction de polymérisation de l'isoprène en utilisant au moins une, sélectionnée, parmi la structure selon l'une quelconque des revendications 1 à 4, et la cellule selon l'une quelconque des revendications 8 à 12 ; et
dans lequel la réaction de polymérisation de l'isoprène est de préférence opérée en utilisant un composé allylique de faible masse moléculaire comme substrat.

14. Procédé de production d'un caoutchouc, le procédé comprenant la production du caoutchouc en utilisant un composé polymérique d'isoprène, dans lequel ledit procédé comprend en outre une étape consistant à opérer une réaction de polymérisation de l'isoprène selon la revendication 13.
